# EUROPEAN PATENT APPLICATION

(11) **EP 4 740 981 A1**
(43) Date of publication of application: **13.05.2026**
(21) Application number: 23948187.2
(22) Date of filing: 07.09.2023
(51) Int. Cl.: A61M 5/158, A61M 5/50

(54) **INDWELLING NEEDLE**

(30) Priority: 04.08.2023 CN 202310976903
(71) Applicant: CMT Health USA LLC, Dover, County of Kent, Delaware 19901 (US); Lu, Jun, Wuxi, Jiangsu 214400 (CN)
(72) Inventor: LU, Jun, Wuxi, Jiangsu 214400 (CN)
(74) Representative: Buzzi, Notaro & Antonielli d'Oulx S.p.A.
(86) International application number: PCT/CN2023/117364
(87) International publication number: WO 2025/030620

(57) **Abstract**

Provided is an indwelling needle, including a needle hub, a catheter hub, and a needle tip protection housing. The needle hub is provided with a needle tube. The catheter hub is provided with a needle piercing channel and an injection side channel. A catheter is disposed at a distal end of the needle piercing channel. An elastic silicone block is disposed in the needle piercing channel. A limiting piece and an elastic piece are disposed in the needle tip protection housing. A first limiting hole allowing the needle tube to pass through is opened on the limiting piece. A second limiting hole allowing the needle tube to pass through is opened on the elastic piece. In an initial state, the needle tube passes through the first limiting hole and the second limiting hole in the needle tip protection housing, and extends into the catheter after piercing the elastic silicone block. In a needle withdrawal state, the elastic silicone block seals a piercing hole of the needle tube by virtue of an elastic force of the elastic silicone block. The elastic piece is deformed by an elastic force of the elastic piece such that the first limiting hole and the second limiting hole are misaligned, and a passage path of the needle tube is blocked.

## Description

This application claims priority to Chinese Patent Application No. 202310976903.2 filed with the China National Intellectual Property Administration (CNIPA) on Aug. 04, 2023, the disclosure of which is incorporated herein by reference in its entirety.

### TECHNICAL FIELD

This application relates to the technical field of medical devices, for example, to an indwelling needle.

### BACKGROUND

An intravenous indwelling needle is a tool for infusion. It commonly consists of a stainless steel needle core, a soft outer hollow cannula, and a plastic needle hub. During a puncture, the outer hollow cannula and the needle core are inserted into a blood vessel together. Thereafter, the needle core is withdrawn, leaving the outer hollow cannula in the blood vessel. For patients requiring long-term infusion or intermittent and continuous infusion, the use of an intravenous indwelling needle can avoid damage and complications caused by repeated punctures. Therefore, the intravenous indwelling needle is widely used in clinical practice.

### SUMMARY

This application provides an indwelling needle including a needle hub, a catheter hub, and a needle tip protection housing. The needle hub is provided with a needle tube. The catheter hub is provided with a needle piercing channel and an injection side channel. A catheter is disposed at a distal end of the needle piercing channel. An elastic silicone block is disposed in the needle piercing channel. A limiting piece and an elastic piece are disposed in the needle tip protection housing. A first limiting hole allowing the needle tube to pass through is opened on the limiting piece. A second limiting hole allowing the needle tube to pass through is opened on the elastic piece. In an initial state, the needle tube passes through the first limiting hole and the second limiting hole in the needle tip protection housing, and extends into the catheter after piercing the elastic silicone block. In a needle withdrawal state, the elastic silicone block seals a piercing hole of the needle tube by virtue of an elastic force of the elastic silicone block. The elastic piece is deformed by an elastic force of the elastic piece such that the first limiting hole and the second limiting hole are misaligned, and a passage path of the needle tube is blocked.

### BRIEF DESCRIPTION OF DRAWINGS

FIG. 1 is a view illustrating the external appearance of an indwelling needle according to an embodiment of this application.
FIG. 2 is an exploded view of an indwelling needle according to an embodiment of this application.
FIG. 3 is a view illustrating the installation of an elastic piece and a limiting piece in a needle tip protection housing of an indwelling needle according to an embodiment of this application.
FIG. 4 is a view of an elastic piece in an indwelling needle according to an embodiment of this application.
FIG. 5 is a view of a limiting piece in an indwelling needle according to an embodiment of this application.
FIG. 6 is a view of a catheter hub rear cover in an indwelling needle according to an embodiment of this application.

### Reference list

- 1: needle hub
- 11: needle tube
- 12: clamping slot
- 13: arrow
- 2: catheter hub
- 21: needle piercing channel
- 22: injection side channel
- 23: catheter
- 24: protrusion
- 3: needle tip protection housing
- 31: limiting block
- 32: insertion slot
- 33: convex rib
- 4: elastic silicone block
- 41: isolation plug
- 42: hollow sleeve
- 5: limiting piece
- 51: first limiting hole
- 6: elastic piece
- 61: second limiting hole
- 62: fixing portion
- 63: V-shaped energy storage portion
- 64: opening portion
- 65: snap portion
- 7: catheter hub rear cover
- 71: relief opening
- 8: cover plate

### DETAILED DESCRIPTION

Indwelling needles are mostly used in a form where the needle is withdrawn after use. After the needle core is withdrawn, the indwelling needle has a risk of leakage due to the needle hole. In addition, the needle core may be reused. In particular, after the needle tip has contacted a patient's blood, the reused needle core may cause infection to external personnel through contact, leading to hazards such as tetanus, bacterial invasion, and infectious disease transmission.

In view of the above circumstances, embodiments of this application provide an indwelling needle.

The embodiments of this application are described in detail below. Examples of the embodiments are shown in the drawings, where the same or similar reference numerals throughout indicate the same or similar components or components having the same or similar functions. The embodiments described below with reference to the drawings are exemplary, intended to explain this application, and not to be construed as limiting this application.

In the description of this application, unless otherwise expressly specified and limited, a term "connected to each other", "connected", or "secured" is to be construed in a broad sense, for example, as securely connected or detachably connected; mechanically connected or electrically connected; indirectly connected to each other via an intermediary; or internally connected between two components or interaction relations between two components. For those of ordinary skill in the art, specific meanings of the preceding terms in this application may be construed according to specific circumstances.

In the description of this application, unless otherwise expressly specified and limited, when a first feature is described as "above" or "below" a second feature, the first feature and the second feature may be in direct contact or be in contact via another feature between the two features instead of being in direct contact. Moreover, when the first feature is "on", "above", or "over" the second feature, the first feature is right on, above, or over the second feature, or the first feature is obliquely on, above, or over the second feature, or the first feature is simply at a higher level than the second feature. When the first feature is "under", "below", or "underneath" the second feature, the first feature is right under, below, or underneath the second feature, or the first feature is obliquely under, below, or underneath the second feature, or the first feature is simply at a lower level than the second feature. Technical solutions of this application are further described hereinafter through embodiments in conjunction with the drawings.

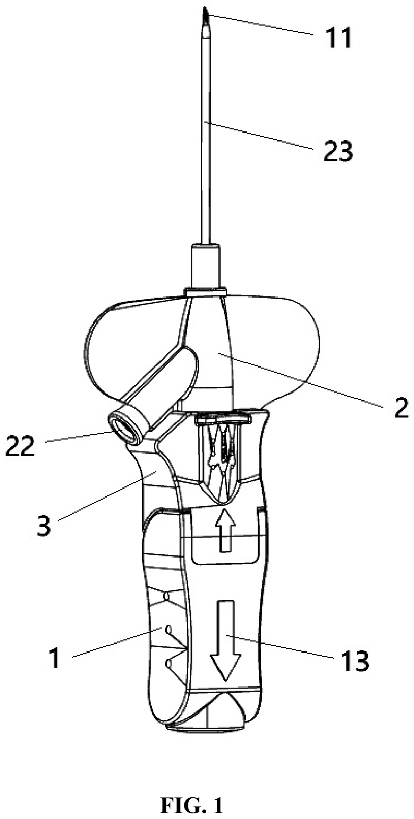

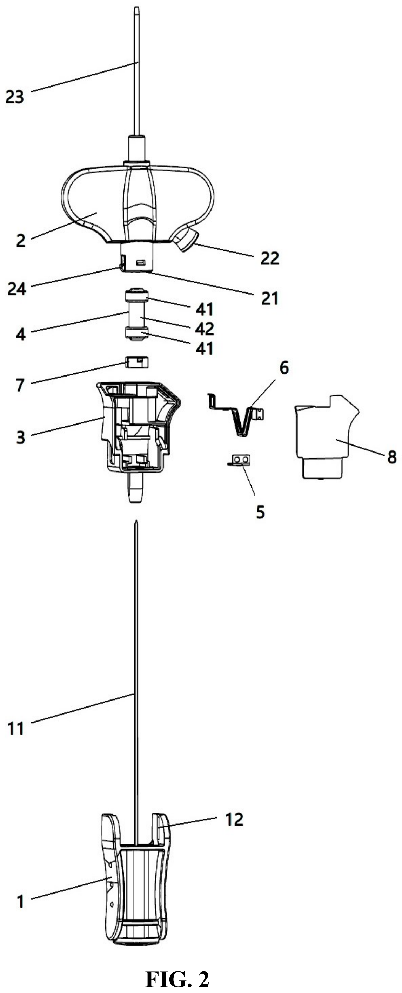

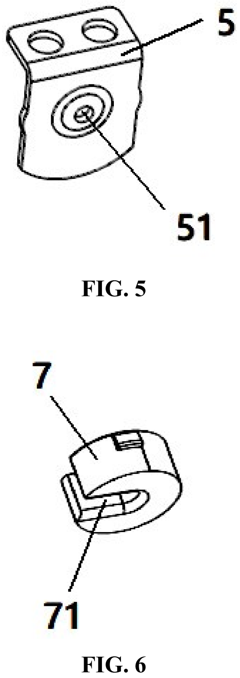

With reference to FIG. 1 to FIG. 6, this embodiment provides an indwelling needle including a needle hub 1, a catheter hub 2, and a needle tip protection housing 3. The needle hub 1 is provided with a needle tube 11. The catheter hub 2 is provided with a needle piercing channel 21 and an injection side channel 22. A catheter 23 is disposed at a distal end of the needle piercing channel 21. An elastic silicone block 4 is disposed in the needle piercing channel 21. A limiting piece 5 and an elastic piece 6 are disposed in the needle tip protection housing 3. A first limiting hole 51 allowing the needle tube 11 to pass through is opened on the limiting piece 5. A second limiting hole 61 allowing the needle tube 11 to pass through is opened on the elastic piece 6.

In an initial state, the needle tube 11 passes through the first limiting hole 51 and the second limiting hole 61 in the needle tip protection housing 3, and extends into the catheter 23 after piercing the elastic silicone block 4.

In a needle withdrawal state, the elastic silicone block 4 seals a piercing hole of the needle tube 11 by virtue of an elastic force of the elastic silicone block 4. The elastic piece 6 is deformed by an elastic force of the elastic piece 6 such that the first limiting hole 51 and the second limiting hole 61 are misaligned, and a passage path of the needle tube 11 is blocked.

In an embodiment, the elastic silicone block 4 includes two isolation plugs 41 arranged at intervals. A hollow sleeve 42 is connected between the two isolation plugs 41. The needle tube 11 passes through one isolation plug 41, then passes through a cavity of the hollow sleeve 42, and then passes out from the other isolation plug 41. The hollow sleeve 42 can reduce resistance to the passage of the needle tube 11. The isolation plugs 41 at two ends improve the sealing performance of the passage path of the needle tube 11, effectively preventing leakage of an injection solution.

In an embodiment, during the needle withdrawal process of the needle tube 11, when the needle tube 11 exits the elastic silicone block 4, the elastic piece 6 deforms and cooperates with the limiting piece 5 to bend the needle tube 11. Thus, on the one hand, deformation of the elastic piece 6 changes the position of the second limiting hole 61 to block the passage path of the needle tube 11. On the other hand, deformation of the elastic piece 6 applies a force to the needle tube 11, causing the needle tube 11 to be bent or even broken at the first limiting hole 51, rendering the needle tube 11 unusable again, and avoiding secondary infection.

In an embodiment, a catheter hub rear cover 7 is disposed at an opening end of the needle piercing channel 21. The catheter hub rear cover 7 axially limits the elastic silicone block 4 to prevent the elastic silicone block 4 from moving out with the needle tube 11 during needle withdrawal and thus avoid a sealing failure. Moreover, a relief opening 71 consistent with a bending direction of the needle tube 11 is opened on the catheter hub rear cover 7, so as to prevent the needle tip from damaging or touching the elastic silicone block 4 or the catheter hub rear cover 7 when the needle tube 11 is bent.

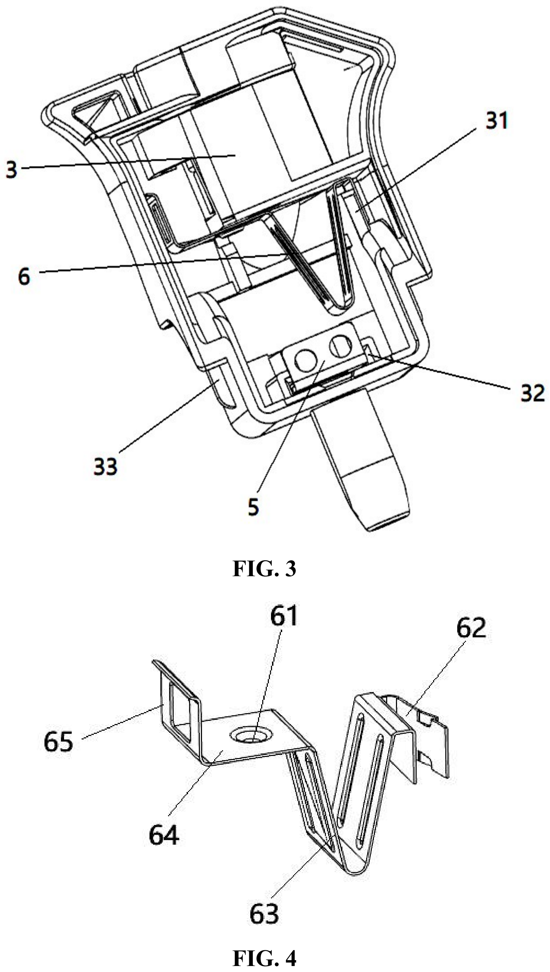

In an embodiment, the elastic piece 6 is integrally formed by bending. With reference to FIG. 4, the elastic piece includes a fixing portion 62, a V-shaped energy storage portion 63, and an opening portion 64 that are sequentially connected. A limiting block 31 for clamping the fixing portion 62 is disposed in the needle tip protection housing 3. The V-shaped energy storage portion 63 exerts a force on the opening portion 64 to repel the opening portion 64 away from the fixing portion 62. The second limiting hole 61 is formed on the opening portion 64 such that the position of the second limiting hole 61 is offset during needle withdrawal, and the energy stored in the V-shaped energy storage portion 63 is sufficiently large, thus a force required to bend the needle tube 11 can be provided.

In an embodiment, the opening portion 64 is provided with a snap portion 65 extending toward the catheter hub 2. The catheter hub 2 is inserted into the needle tip protection housing 3, and an insertion portion of the catheter hub 2 is provided with a protrusion 24. The snap portion 65 engages with the protrusion 24, thereby achieving structural stability of the components in the initial state.

In an embodiment, the limiting piece 5 is located at an entry for the needle tube 11 on the needle tip protection housing 3. The needle tip protection housing 3 is provided with an insertion slot 32 allowing insertion of the limiting piece 5, thereby facilitating the accurate installation of the limiting piece 5 in the needle tip protection housing 3.

In an embodiment, one side surface of the needle tip protection housing 3 is open, and a cover plate 8 connected by snap-fit is disposed on the open surface. This configuration facilitates assembly and inspection in case of abnormalities. The cover plate 8 can also prevent blood on the needle tip from leaking out and prevent the needle tip from being manually pushed out again.

In addition, the needle hub 1 is fixed to the needle tip protection housing 3 by a plug-in connection. The plug-in structures of the two may be interchanged. Here, a convex rib 33 is disposed on an insertion portion of the needle tip protection housing 3. A clamping slot 12 corresponding to the convex rib 33 is disposed on the needle hub 1. Arrows 13 guiding separation are marked on the needle hub 1 and the needle tip protection housing 3, respectively, which facilitates correct use and convenient needle withdrawal.

In summary, the preceding indwelling needle achieves reliable sealing after needle withdrawal by means of the elastic silicone block. Furthermore, with the elastic piece and the limiting piece in the needle tip protection housing, the passage path of the needle tube can be blocked, or even the needle tube can be damaged. Thus, reuse of the puncture needle is prevented, leakage, secondary infection, and other usage conditions are avoided, and the safety of use of the indwelling needle is improved.

The indwelling needle provided by the embodiments of this application improves its safety and convenience of use, and avoids blood overflow and reuse of the needle core.

The embodiments of this application also encompass various variations and modifications, all of which fall within the scope of this application as claimed.

## Claims

1. An indwelling needle, comprising a needle hub (1), a catheter hub (2), and a needle tip protection housing (3), wherein the needle hub (1) is provided with a needle tube (11), the catheter hub (2) is provided with a needle piercing channel (21) and an injection side channel (22), and a catheter (23) is disposed at a distal end of the needle piercing channel (21), wherein
an elastic silicone block (4) is disposed in the needle piercing channel (21), a limiting piece (5) and an elastic piece (6) are disposed in the needle tip protection housing (3), a first limiting hole (51) allowing the needle tube (11) to pass through is opened on the limiting piece (5), and a second limiting hole (61) allowing the needle tube (11) to pass through is opened on the elastic piece (6);
in an initial state, the needle tube (11) passes through the first limiting hole (51) and the second limiting hole (61) in the needle tip protection housing (3), and extends into the catheter (23) after piercing the elastic silicone block (4); and
in a needle withdrawal state, the elastic silicone block (4) seals a piercing hole of the needle tube (11) by virtue of an elastic force of the elastic silicone block (4), and the elastic piece (6) is deformed by an elastic force of the elastic piece (6) such that the first limiting hole (51) and the second limiting hole (61) are misaligned, and a passage path of the needle tube (11) is blocked.

2. The indwelling needle according to claim 1, wherein the elastic silicone block (4) comprises two isolation plugs (41) arranged at intervals, a hollow sleeve (42) is connected between the two isolation plugs (41), and the needle tube (11) passes through one isolation plug (41) of the two isolation plugs (41), then passes through a cavity of the hollow sleeve (42), and then passes out from the other isolation plug (41) of the two isolation plugs (41).

3. The indwelling needle according to claim 1, wherein the elastic piece (6) comprises a fixing portion (62), a V-shaped energy storage portion (63), and an opening portion (64) that are sequentially connected, a limiting block (31) for clamping the fixing portion (62) is disposed in the needle tip protection housing (3), the V-shaped energy storage portion (63) exerts a force on the opening portion (64) to repel the opening portion (64) away from the fixing portion (62), and the second limiting hole (61) is formed on the opening portion (64).

4. The indwelling needle according to claim 3, wherein the opening portion (64) is provided with a snap portion (65) extending toward the catheter hub (2), the catheter hub (2) is inserted into the needle tip protection housing (3), an insertion portion of the catheter hub (2) is provided with a protrusion (24), and the snap portion (65) engages with the protrusion (24).

5. The indwelling needle according to claim 1, wherein the limiting piece (5) is located at an entry for the needle tube (11) on the needle tip protection housing (3), and the needle tip protection housing (3) is provided with an insertion slot (32) allowing insertion of the limiting piece (5).

6. The indwelling needle according to claim 1, wherein during a needle withdrawal process of the needle tube (11), in a case where the needle tube (11) exits the elastic silicone block (4), the elastic piece (6) deforms and cooperates with the limiting piece (5) to bend the needle tube (11).

7. The indwelling needle according to claim 6, wherein a catheter hub rear cover (7) is disposed at an opening end of the needle piercing channel (21), the catheter hub rear cover (7) axially limits the elastic silicone block (4), and a relief opening (71) consistent with a bending direction of the needle tube (11) is opened on the catheter hub rear cover (7).

8. The indwelling needle according to claim 1, wherein one side surface of the needle tip protection housing (3) is open, and a cover plate (8) connected by snap-fit is disposed on the open surface.

9. The indwelling needle according to claim 1, wherein the needle hub (1) is fixed to the needle tip protection housing (3) by a plug-in connection, an insertion portion of one of the needle hub (1) and the needle tip protection housing (3) is provided with a convex rib (33), and the other of the needle hub (1) and the needle tip protection housing (3) is provided with a clamping slot (12) corresponding to the convex rib (33).

10. The indwelling needle according to claim 9, wherein the needle hub (1) and the needle tip protection housing (3) are marked with arrows (13) guiding separation, respectively.
